# EUROPÄISCHE PATENTSCHRIFT

(11) **EP 0 670 303 B1**
(45) Veröffentlichungstag und Bekanntmachung des Hinweises auf die Patenterteilung: **14.10.1998**
(21) Anmeldenummer: 95102327.4
(22) Anmeldetag: 20.02.1995
(51) Int. Cl.: C07C 249/02

(54) **Verfahren zur Herstellung von aliphatischen Iminen**
Process for the preparation of aliphatic imines
Procédé de préparation d'imines aliphatiques

(30) Priorität: 03.03.1994 DE 4406949
(43) Veröffentlichungstag der Anmeldung: 06.09.1995
(73) Patentinhaber: BAYER AG, 51368 Leverkusen (DE)
(72) Erfinder: Lantzsch, Reinhard, Dr., D-42115 Wuppertal (DE); Lindner, Werner, Dr., D-51067 Köln (DE)

(56) Entgegenhaltungen:
- EP-A- 0 006 180
- US-A- 2 582 128
- TETRAHEDRON, Bd.36, Nr.1, 1980, OXFORD GB R. VERHE 'Reactions of N-1(2,2-Dichloroalkylidene)amines with Potassium Cyanides: Synthesis of .beta.-Chloro-.alpha.-cyanoenamines, .alpha.-Chloroimidates and 2-Amino-5-cyanopyrrols'
- CHEMISTRY LETTERS, 1992, TOKYO JP Seiten 1493 - 1496 M. FUJII ET AL. 'Stereoselective Synthesis of (+-)-Isonitramine and (+-)-Sibirine'

## Beschreibung

Die Erfindung betrifft ein neues Verfahren zur Herstellung von aliphatischen Iminen, welche als Zwischenprodukte zur Herstellung von agrochemischen oder pharmazeutischen Wirkstoffen verwendet werden können.

Es ist bekannt, daß im Gegensatz zur Synthese aromatischer Imine die Herstellung aliphatischer Imine relativ schwierig ist (vgl. Chem. Reviews 63 (1963), 489-510, insbesondere 493).

Als einzige präparative Methode zur Herstellung aliphatischer Imine - in mäßigen Ausbeuten - ist die lösungsmittelfreie Umsetzung von Aldehyden mit Aminen in Gegenwart von festem Kaliumhydroud bekannt (vgl. J. Am. Chem. Soc. 66 (1944), 82-84; J. Chem. Soc. Perkin Trans. I 1984, 1173-1182). Diese Methode ist für den industriellen Bedarf wenig geeignet, da Ausbeute und Qualität der Produkte in vielen Fällen unbefriedigend sind, die Nebenprodukte nur schwierig abzutrennen sind und große Mengen Kaliumhydroxid zu entsorgen sind.

Die Umsetzung von Aldehyden mit Aminen zu Aldiminen ist zwar grundsätzlich bekannt, z.T. aus Tetrahedron Vol. 36, p 131 - 142 (1980); Chemistry letters pp 1493 - 1496 (1992); EP-A 6 180; US-P 2 582 128. Es werden jedoch in diesem Stand der Technik keine Angaben zu Reaktionsbedingungen gemacht unter denen in technischem Maßstab unverzweigte aliphatische Aldehyde mit Aminen in guter Ausbeute umgesetzt werden können.

Die vorliegende Erfindung betrifft nun das Verfahren zur Herstellung von aliphatischen Iminen der allgemeinen Formel (I) 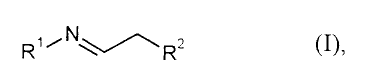 in welcher
- R¹: für jeweils gegebenenfalls durch Fluor, Chlor, Brom, Methyl, Ethyl, n- oder i-Propyl, n-, i-, S- oder t-Butyl, Methoxy oder Ethoxy substituiertes Benzyl, Phenylethyl, Phenylpropyl steht, und
- R²: für jeweils gegebenenfalls durch Fluor, Chlor, Methoxy oder Ethoxy substituiertes Methyl, Ethyl, n-Propyl, n-Butyl steht,
das dadurch gekennzeichnet ist, daß man
Amine der allgemeinen Formel (II)

R¹-NH₂ (II)

in welcher
- R¹: die oben angegebene Bedeutung hat,
mit Aldehyden der allgemeinen Formel (III) 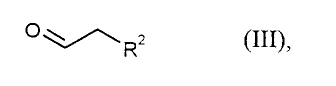 in welcher
- R²: die oben angegebene Bedeutung hat,
in Gegenwart eines mit Wasser praktisch nicht mischbaren organischen Lösungsmittels aus der Reihe der aromatischen Kohlenwasserstoffe bei Temperaturen von -30°C bis 15°C im Molverhältnis Amin zu Aldehyd wie 1 : (0,95 - 1,05) umsetzt.

Überraschendeiweise können nach dem erfindungsgemäßen Verfahren die Imine der Formel (I) ohne Zusatz eines festen Hilfsstoffes in praktisch quantitativen Ausbeuten erhalten werden.

Das erfindungsgemäße Verfahren stellt somit eine wertvolle Bereicherung des Standes der Technik dar.

Verwendet man beispielsweise Methylamin und Propionaldehyd als Ausgangsstoffe, so kann der Reaktionsablauf beim erfindungsgemäßen Verfahren durch das folgende Formelschema skizziert werden:

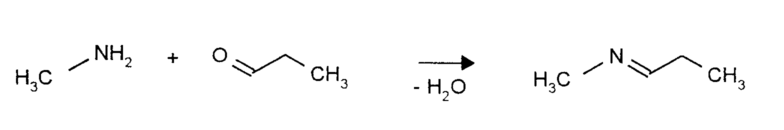

Die beim erfindungsgemäßen Verfahren zur Herstellung der Verbindungen der allgemeinen Formel (I) als Ausgangsstoffe zu verwendenden Amine sind durch die Formel (II) allgemein definiert. In der Formel (II) hat R¹ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R¹ angegeben wurde.

Die Ausgangsstoffe der Formel (II) sind bekannte Synthesechemikalien.

Die beim erfindungsgemäßen Verfahren weiter als Ausgangsstoffe zu verwendenden Aldehyde sind durch die Formel (III) allgemein definiert. In der Formel (II) hat R² vorzugsweise bzw. insbesondere diejenige Bedeutung, die bereits oben im Zusammenhang mit der Beschreibung der Verbindungen der Formel (I) als bevorzugt bzw. als insbesondere bevorzugt für R² angegeben wurde.

Die Ausgangsstoffe der Formel (III) sind bekannte Synthesechemikalien.

Das erfindungsgemäße Verfahren wird in Gegenwart eines mit Wasser praktisch nicht mischbaren organischen Lösungsmittels durchgeführt. Hierzu gehören insbesondere aliphatische, alicyclische oder aromatische, gegebenenfalls halogenierte Kohlenwasserstoffe, wie beispielsweise Benzin, Benzol, Toluol, Xylol, Mesitylen, Chlorbenzol, Dichlorbenzol, Petrolether, Pentan, Hexan, Heptan, Cyclohexan, Methylcyclohexan, Dichlormethan, Chloroform und Tetrachlormethan; Ether, wie beispielsweise Diethylether, Diisopropylether, t-Butyl-methylether, t-Amyl-methylether, Dioxan, Tetrahydrofuran und Ethylenglykoldimethyl- oder -diethylether. Bevorzugt sind aromatische Kohlenwasserstoffe.

Die Reaktionstemperaturen können bei der Durchführung des erfindungsgemäßen Verfahrens in einem größeren Bereich variiert werden. Im allgemeinen arbeitet man bei Temperaturen zwischen -30°C und +15°C, vorzugsweise bei Temperaturen zwischen 0°C und +15°C, insbesondere bei Temperaturen zwischen 5°C und 15°C.

### Herstellungsbeispiel (allgemeine Beschreibung):

1 Mol Aldehyd der Formel (III) wird zu 1 Mol Amin der Formel (II) in 500 ml Toluol getropft. Die Mischung wird dann bei 5°C bis 15°C stehen gelassen, bis die Wasserabscheidung beendet ist. Dann wird die organische Phase abgetrennt, mit Natriumsulfat getrocknet und filtriert.

Das Filtrat enthält das Produkt der Formel (I) in praktisch quantitativer Ausbeute und kann direkt für weitere Umsetzungen verwendet werden.

Auf die oben beschriebene Weise können beispielsweise die in der nachstehenden Tabelle 1 aufgeführten Verbindungen der Formel (I) hergestellt werden.

**Tabelle 1**

| Beispiel für die erfindungsgemäß herzustellenden Verbindungen der Formel (I) | | |
|---|---|---|
| **Bsp.-Nr.** | **R**^{**1**} | **R**^{**2**} |
| 1 | C₆H₅CH₂ | CH₃ |
| 2 | C₆H₅CH₂ | CH₂OCH₃ |
| 3 | C₆H₅CH₂ | C₂H₅ |

Ausbeute und Reinheit der Imine können durch weitere Umsetzung, z.B. katalytische Hydrierung zu sekundären Aminen bestimmt werden.

## Patentansprüche

1. Verfahren zur Herstellung von aliphatischen Iminen der allgemeinen Formel (I) 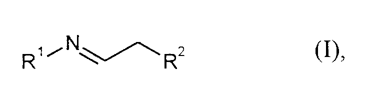 in welcher
R¹ für jeweils gegebenenfalls durch Fluor, Chlor, Brom, Methyl, Ethyl, n- oder i-Propyl, n-, i-, S- oder t-Butyl, Methoxy oder Ethoxy substituiertes Benzyl, Phenylethyl, Phenylpropyl steht, und
R² für jeweils gegebenenfalls durch Fluor, Chlor, Methoxy oder Ethoxy substituiertes Methyl, Ethyl, n-Propyl, n-Butyl steht,
dadurch gekennzeichnet, daß man
Amine der allgemeinen Formel (II)
R¹-NH₂ (II)
in welcher
R¹ die oben angegebene Bedeutung hat,
mit Aldehyden der allgemeinen Formel (III) 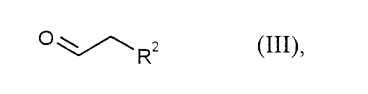 in welcher
R² die oben angegebene Bedeutung hat,
in Gegenwart eines mit Wasser praktisch nicht mischbaren organischen Lösungsmittels aus der Reihe der aromatischen Kohlenwasserstoffe bei Temperaturen von -30°C bis 15°C im Molverhältnis Amin zu Aldehyd wie 1 : (0,95 - 1,05) umsetzt.

## Claims

1. Process for the preparation of aliphatic imines of the general formula (I) 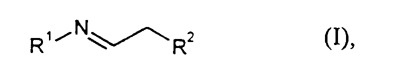 in which
R¹ is benzyl, phenylethyl or phenylpropyl, each of which is optionally substituted by fluorine, chlorine, bromine, methyl, ethyl, n- or i-propyl, n-, i-, s- or t-butyl, methoxy or ethoxy, and
R² is methyl, ethyl, n-propyl or n-butyl, each of which is optionally substituted by fluorine, chlorine, methoxy or ethoxy,
characterized in that
amines of the general formula (II)
R¹-NH₂ (II)
in which
R¹ is as defined above,
are reacted with aldehydes of the general formula (III) 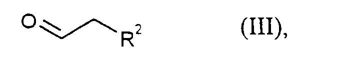 in which
R² is as defined above,
in the presence of a practically water-immiscible organic solvent from the series consisting of the aromatic hydrocarbons at temperatures of from -30°C to 15°C in a molar ratio of amine to aldehyde of 1 : (0.95 - 1.05).

## Revendications

1. Procédé pour la préparation d'imines aliphatiques répondant à la formule générale (I) 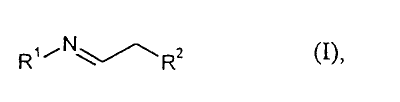 dans laquelle
R¹ représente un groupe benzyle, un groupe phényléthyle, un groupe phénylpropyle portant respectivement, le cas échéant, un ou plusieurs substituants identiques ou différents fluoro, chloro, bromo, méthyle, éthyle, n- ou i-propyle, n-, i-, s- ou t-butyle, méthoxy ou éthoxy, et
R² représente un groupe méthyle, un groupe éthyle, un groupe n-propyle, un groupe n-butyle portant respectivement, le cas échéant, un ou plusieurs substituants identiques ou différents fluoro, chloro, méthoxy ou éthoxy,
caractérisé en ce qu'on fait réagir des amines répondant à la formule générale (II)
R¹-NH₂ (II)
dans laquelle
R¹ a la signification indiquée ci-dessus,
avec des aldéhydes répondant à la formule (III) 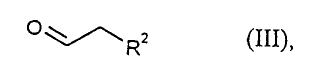 dans laquelle
R² a la signification indiquée ci-dessus,
en présence d'un solvant organique pratiquement non miscible à l'eau choisi parmi la série des hydrocarbures aromatiques, à des températures de -30°C à 15°C, dans un rapport molaire de l'amine à l'aldéhyde de 1:(0,95-1,05).
